# FASCICULE DE BREVET EUROPEEN

(11) **EP 0 538 352 B1**
(45) Date de publication et mention de la délivrance du brevet: **31.01.1996**
(21) Numéro de dépôt: 91913032.8
(22) Date de dépôt: 11.07.1991
(51) Int. Cl.: C12N 15/12, C07K 14/00, C07K 7/08, A61K 39/12, G01N 33/569, C12P 21/08

(54) **SEQUENCE PEPTIDIQUE IMMUNOGENE D'$i(ECHINOCOCCUS GRANULOSUS), SEQUENCE D'ADN CODANT POUR CETTE SEQUENCE PEPTIDIQUE ET APPLICATIONS DIAGNOSTIQUES ET THERAPEUTIQUES**
IMMUNOGENISCHES PEPTID VON ECHINOCOCCUS GRANULOSIS, DAFÜR KODIERENDE DNS SOWIE DIAGNOSTISCHE UND THERAPEUTISCHE VERWENDUNGEN
IMMUNOGENIC PEPTIDE SEQUENCE FROM $i(ECHINOCOCCUS GRANULOSUS), DNA SEQUENCE CODING FOR SAID PEPTIDE SEQUENCE, AND DIAGNOSTIC AND THERAPEUTIC USES THEREOF

(30) Priorité: 12.07.1990 FR 9008900
(43) Date de publication de la demande: 28.04.1993
(73) Titulaire: INSTITUT PASTEUR, F-75724 Paris Cédex 15 (FR); INSTITUT PASTEUR DE LILLE, F-59019 Lille Cédex (FR); INSTITUT NATIONAL DE LA SANTE ET DE LA RECHERCHE MEDICALE (INSERM), F-75654 Paris Cédex 13 (FR)
(72) Inventeur: FACON, Brigitte, F-60200 Compiègne (FR); CHAMEKH, Mustapha, F-59800 Lille (FR); DISSOUS, Colette, F-59262 Sainghin-en-Mélantois (FR); CAPRON, André, F-59133 Phalempin (FR); TARTAR, André, F-62490 Vitry-en-Artois (FR); GRAS MASSE, Hélène, F-59710 Mérignies (FR)
(74) Mandataire: Polus, Camille
(86) Numéro de dépôt international: FR9100563
(87) Numéro de publication internationale: WO9201051

(56) Documents cités:
- Molecular and Biochemical Parasitology, vol. 45, 1991, Elsevier Science Publishers B.V. (Biomedical Division), B. Facon et al.: "Molecular cloning of an Echinococcus granulosus protein expressing an immunogenic epitope of antigen 5", pages 233-240, voir l'article en entier
- Molecular and Biochemical Parasitology, vol. 20, 1986, Elsevier Science Publishers B.V. (Biomedical Division), G. Di Felice et al.: "Purification and partial characterization of the major antigen of Echinoccocus granulosus (antigen 5) with monoclonal antibodies", pages 133-142, voir l'article en entier
- Biological Abstracts, vol. 88, 1989, Biological Abstracts, Inc., (Philadelphia, PA, US), B. Hamrioui et al.: "Production of anti-hydatid monoclonal antibodies", voir résumé 120220, & Arch. Inst. Pasteur Alger. 56 (0), 1988 (1989), 130-146
- Molecular and Biochemical Parasitology, vol. 36, 1989, Elsevier Science Publishers B.V. (Biomedical Division), M.W. Lightowlers et al.: "Amino acid sequence homology between cyclophilin and a cDNA-cloned antigen of Echinococcus granulosus", pages 287-290, voir l'article en entier
- Molecular and Biochemical Parasitology, vol. 33, 1989, Elsevier Science Publishers B.V. (Biomedical Division), L. Hemmings et al.: "The isolation, by differential antibody screening, of Echinococcus multilocularis antigen gene clones with potential for immunodiagnosis", pages 171-182

## Description

La présente invention se rapporte à une séquence peptidique immunogène d'Echinococcus granulosus, une séquence d'ADN codant pour cette séquence peptidique ainsi que les applications diagnostiques et thérapeutiques de la séquence peptidique.

L'hydatidose ou échinococcose uniloculaire est une maladie parasitaire due au développement chez l'homme ainsi que le bétail de la forme larvaire d'un petit cestode, Echinococcus granulosus. Le chien représente le réservoir du parasite adulte. Cette parasitose est un véritable fléau dans les pays d'élevage en Afrique, en Amérique Latine et en Australie ainsi que les pays du pourtour du bassin méditerranéen où existent des répercussions économiques à la fois dans le domaine médical et vétérinaire. Si aujourd'hui l'on ne dispose pratiquement pas de test de diagnostic pour l'animal, le diagnostic médical est souvent délicat à cause de la non spécificité des symptômes observés. En revanche, plusieurs méthodes immunologiques classiques tel l'hémagglutination et la réaction de fixation du complément ont été pratiquées pour le dépistage de l'hydatidose. Le liquide hydatique, utilisé comme source d'antigènes, contient en plus des produits de métabolisme du parasite communs à d'autres helminthes, plusieurs composants de l'hôte (CAPRON A. et al (1), RUSSI S. et al (2), BEN-ISMAEL R. et al (3)). Cela pose le problème des réactions faussement positives. Cependant, il a été montré par la technique d'immunoélectrophorèse, la présence presque constante d'anticorps précipitant un antigène désigné antigène 5 (CAPRON A. et al (4) et (5)). Depuis, ce test demeure l'un des meilleurs tests immunologiques de référence pour toute étude épidémiologique de l'hydatidose. L'antigène 5, très immunogène, a fait l'objet de plusieurs travaux montrant qu'il s'agit d'une lipoprotéine capable de se fixer à la concanavaline A, thermolabile, de masse moléculaire relative d'environ 60 kDa (ORIOL R. et al (6), BOUT D. et al (7), PIANTELLI M. (8)). Analysé dans des conditions réductrices, cet antigène se dissocie en deux sous-unités de 37 et 22 KDa. En utilisant des techniques plus sensibles telles que l'ELISA, les potentialités diagnostiques de l'antigène 5 purifié ont été largement confirmées. Des anticorps anti antigène 5 ont été aussi détectés dans des sérums de patients ou d'animaux infectés par E. multilocularis ou par d'autres cestodes tels que Taenia hydatigena, T. ovis et T. solium. Cependant leur taux reste faible. Ces quelques réactions faussement positives observées ont été attribuées en partie à la présence d'un épitope phosphorylcholine sur l'antigène 5.

Le problème actuel est de pouvoir disposer d'antigènes parasitaires spécifiques, bien standardisés et en quantité suffisante pour les tests de diagnostic. C'est dans cette optique que les travaux actuellement menés sur l'hydatidose et qui sont basés sur les techniques des anticorps monoclonaux et de génie génétique cherchent à isoler et à identifier des composants parasitaires possédant un fort degré de spécificité.

La présente invention a pour but de fournir un antigène parasitaire spécifique de l'hydatidose.

Les inventeurs ont caractérisé un anticorps monoclonal d'isotype IgG₁ désigné EG 02 154/12 dirigé contre un épitope protéique porté par l'antigène 5, et spécifique d'E. granulosus.

Cet anticorps monoclonal a permis d'isoler dans une banque d'ADN complémentaire obtenue à partir d'ARNm de protoscolex d'E. granulosus des clones désignés Eg 6 et Eg 14 correspondant à des séquences peptidiques qui se sont avérées être reconnues par l'anticorps monoclonal EG 02 154/12.

La présente invention a, par conséquent, pour objet une séquence peptidique immunogène de l'antigène 5 d'Echinococcus granulosus caractérisée en ce qu'elle est reconnue à la fois par des sérums de patients atteints d'hydatidose et par un anticorps monoclonal d'isotype IgG₁ produit par l'hybridome EG 02 154/12 déposé à la CNCM le 25 juin 1990 sous le n° 1-957.

La présente invention a également pour objet une séquence d'ADN comprenant la séquence nucléotidique désignée Eg 6,

10 20 30 40 50 60
GAATTCGTAGACATCAACATTGCATCTAAAGTCGCGGATGCTTTCCAGAAGAATAAGGAG

70 80 90 100 110 120
AAGATTACTACTACCGACAAACTGGGTACTGCTCTCGAGCAGGTTGCTTCCCAATCAGAA

130 140 150 160 170 180
AAGGCAGCTCCCCAACTTTCTAAAATGCTGACGGAAGCTTCTGATGTCCATCAGCGTATG

190 200 210 220 230 240
GCCACTGCCAGAAAGAATTTCAATAGTGAGGTTAATACCACCTTCATTGAAGATTTGAAA

250 260 270 280 290 300
AACTTCTTGAACACCACGCTTAGCGAGGCCCAGAAAGCAAAGACCAAGCTGGAGGAGGTT

310 320 330 340 350 360
CGACTAGATTTGGACTCTGACAAGACTAAATTGAAGAATGCTAAGACTGCGGAACAGAAG

370 380 390 400 410 420
GCCAAGTGGGAGGCCGAGGTGCGAAAAGACGAAAGTGACTTCGATCGAGTGCACCAAGAA

430 440 450
TCTCTTACTATCTTTGAGAAGACTTGCAAAGAATTC

La présente invention a également pour objet la séquence peptidique immunogène de l'antigène 5 d'E. granulosus désignée séquence peptidique Eg 6 codée par la séquence nucléotidique ci-dessus comprenant tout ou partie de la séquence 
GluPheValAspIleAsnIleAlaSerLysValAlaAspAlaPheGlnLysAsnLysGlu

LysIleThrThrThrAspLysLeuGlyThrAlaLeuGluGlnValAlaSerGlnSerGlu

LysAlaAlaProGlnLeuSerLysMETLeuThrGluAlaSerAspValHisGlnArgMET

AlaThrAlaArgLysAsnPheAsnSerGluValAsnThrThrPheIleGluAspLeuLys

AsnPheLeuAsnThrThrLeuSerGluAlaGlnLysAlaLysThrLysLeuGluGluVal

ArgLeuAspLeuAspSerAspLysThrLysLeuLysAsnAlaLysThrAlaGluGlnLys

AlaLysTrpGluAlaGluValArgLysAspGluSerAspPheAspArgValHisGlnGlu

SerLeuThrIlePheGluLysThrCysLysGluPhe
 ainsi que les séquences qui en diffèrent par un ou plusieurs amino-acides et qui possèdent une activité immunogène analogue.

A partir de la séquence du clone Eg 6, on peut envisager la synthèse de 13 peptides dont les séquences correspondent respectivement aux amino-acides 1 à 25, 12 à 34, 22 à 44, 35 à 48, 41 à 63, 46 à 65, 60 à 91, 66 à 96, 89 à 122, 93 à 115, 98 à 129, 118 à 152 et 130 à 152 et sont les suivantes :

EFVDINIASKVADAFQKNKEKITTT
ADAFQKNKEKITTTDKLGTALEQ
ITTTDKLGTALEQVASQSEKAAP
VASQSEKAAPQLSK
KAAPQLSKMLTEASDVHQRMATA
LSKMLTEASDVHQRMATARK
MATARKNFNSEVNTTFIEDLKNFLNTTLSEAQ
NFNSEVNTTFIEDLKNFLNTTLSEAQKAKTK
EAQKAKTKLEEVRLDLDSDKTKLKNAKTAEQKAK
AKTKLEEVRLDLDSDKTKLKNAK
EEVRLDLDSDKTKLKNAKTAEQKAKWEAEVRK
EQKAKWEAEVRKDESDFDRVHQESLTIFEKTCKEF
KDESDFDRVHQESLTIFEKTCKEF

On préfère en particulier la séquence 89 à 122 :

EAQKAKTKLEEVRLDLDSDKTKLKNAKTAEQKAK

Le peptide correspondant dénommé 89/122 est particulièrement intéressant car il présente une forte homologie au niveau de la structure secondaire (hélice α ) avec la séquence peptidique obtenue à partir d'un clone Eg 14 sélectionné à partir de la banque d'ADNc de E. granulosus, dont la séquence nucléotidique est la suivante :

10 20 30 40
GAA TTC ATT CGA AAG TAT GAC AAG GGC AAT AAA GGC AAG ATC AAC
50 60 70 80 90
TTG GAA GAG TTG ACT GCT ATG CTC GAC AGT GTT CAT AGA AAA ACC
100 110 120 130
AGT AGA GCC TCA ATG AGC CGA TGA AGC ATT TAA AAT TAT GAG AAT

Un autre objet de l'invention est également une séquence d'ADN comprenant la séquence du clone EG 14, ci-dessus.

L'invention a également pour objet la séquence peptidique immunogène de l'antigène 5 d'E. granulosus codée par Eg 14 comprenant tout ou partie de la séquence 
E F I R K Y D K G N K G K I N
L E E L T A M L D S V H R K T
S R A S M S R
 ainsi que les séquences qui en diffèrent par un ou plusieurs amino-acides et qui possèdent une activité immunogène analogue.

Dans la présente invention, les inventeurs ont tout d'abord obtenu un ADN complémentaire Eg 6 par les opérations suivantes :
- extraction de l'ARN des protoscolex d'E. granulosus,
- obtention de l'ADN complémentaire simple brin à partir de l'ARN messager, puis de l'ADN complémentaire double-brin,
- insertion dans un vecteur tel que le bactériophage λgt11,
- criblage de la banque et sélection de clones recombinants positifs,
- transfert de l'ADNc sélectionné dans un vecteur phagique pour l'amplifier, le purifier et en réaliser le séquençage.

Les séquences peptidiques selon l'invention peuvent être obtenues par synthèse peptidique classique, par utilisation de la méthode Applied Biosystems ou par application de techniques de génie génétique comprenant l'insertion d'une séquence d'ADN codant pour une séquence peptidique selon l'invention dans un vecteur d'expression tel qu'un plasmide et la transformation de cellules avec ce vecteur d'expression.

La présente invention a donc également pour objet des plasmides et des vecteurs d'expression comprenant une séquence d'ADN codant pour une séquence peptidique selon l'invention ainsi que des hôtes transformés avec ce vecteur.

La présente invention a également pour objet un procédé de diagnostic in vitro de l'hydatidose chez l'homme ou chez l'animal, par mise en évidence des anticorps dirigés contre une séquence peptidique immunogène telle que définie précédemment, dans un prélèvement biologique de l'homme ou de l'animal dans lequel on met en contact la ou les séquences peptidiques immunogènes avec le prélèvement biologique de l'homme ou de l'animal pouvant contenir lesdits anticorps et on révèle la présence des anticorps fixés.

Ce procédé peut être basé sur une méthode radioimmunologique de type RIA, RIPA ou IRMA ou une méthode immunoenzymatique de type WESTERN-BLOT sur bandelettes ou de type ELISA.

La présente invention a également pour objet une trousse de diagnostic in vitro de l'hydatidose pour la mise en oeuvre du procédé ci-dessus mentionné, comprenant au moins une séquence peptidique immunogène telle que décrite précédemment, et contenant en outre un anticorps spécifique d'un isotype d'immunoglobuline.

La présente invention a également pour objet un vaccin destiné au traitement et à la prévention de l'hydatidose chez l'homme ou l'animal dans lequel l'agent vaccinant consiste en une séquence peptidique immunogène telle que définie précédemment.

Outre la séquence peptidique immunogène, le vaccin peut contenir un adjuvant doué de propriétés immunostimulantes.

Parmi les adjuvants que l'on peut utiliser figurent les sels minéraux tels que l'hydroxyde d'aluminium, des composés hydrophobes ou des surfactants tels que l'adjuvant incomplet de Freund, le squalane ou des liposomes, des polynucléotides synthétiques, des microorganismes ou composants de microorganismes tels que le murabutide, des molécules artificielles synthétiques telles que l'imuthiol ou le levamisole, ou encore des cytokines telles que les interférons α, β, γ ou les interleukines.

L'invention a en outre pour objet un anticorps monoclonal dirigé contre une séquence peptidique immunogène telle que définie précédemment.

Les anticorps monoclonaux selon l'invention peuvent être préparés selon une technique classique. A cet effet, les polypeptides peuvent être couplés si nécessaire à un agent immunogène, tel que l'anatoxine tétanique, par un agent de couplage tel que le glutaraldéhyde, un carbodiimide ou la benzidine bis diazotée.

On préfère, en particulier, l'anticorps monoclonal de la sous-classe Ig G₁ obtenu à partir de l'hybridome EG 02 154/12 déposé à la CNCM le 25 juin 1990 sous le n° 1-957 qui constitue également un objet de l'invention.

On décrira ci-après plus en détail :
- l'obtention de l'anticorps EG 02 154/12
- l'obtention de l'ADNc Eg 6 et de l'ADNc Eg 14 codant pour les séquences peptidiques selon l'invention, en se référant aux dessins annexés sur lesquels :

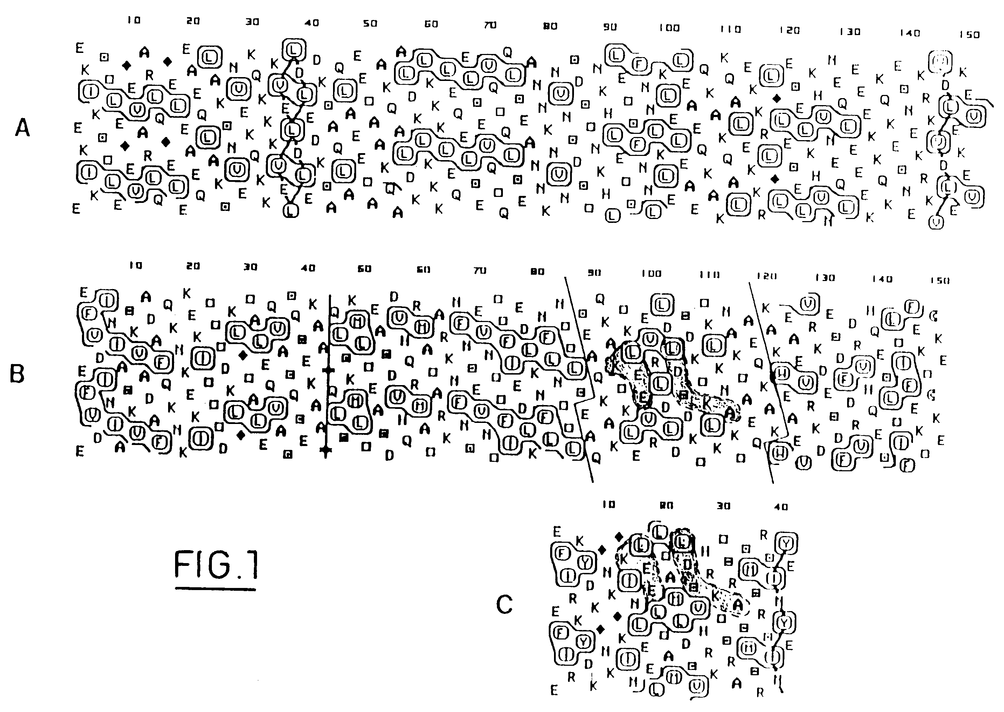

- la Fig. 1 illustre la représentation obtenue après analyse des groupes hydrophobes (Hydrophobic Cluster Analysis (HCA)) de la chaîne lourde de la myosine de muscle squelettique de lapin (A), et les protéines recombinantes Eg6 (B) et Eg14 (C). Les résidus hydrophobes adjacents sont entourés. Certains résidus sont représentés par les symboles : (P(*) G(◆) T(□) S( 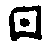 )). L'homologie de motifs topographiques entre Eg6 et Eg14 est en traits hachurés;

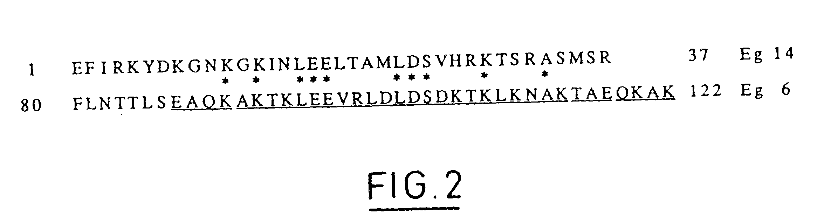

- la Fig. 2 représente les homologies entre la séquence d'aminoacides du clone Eg14 et celle du clone Eg6. Les aminoacides identiques sont indiqués par un astérisque ;

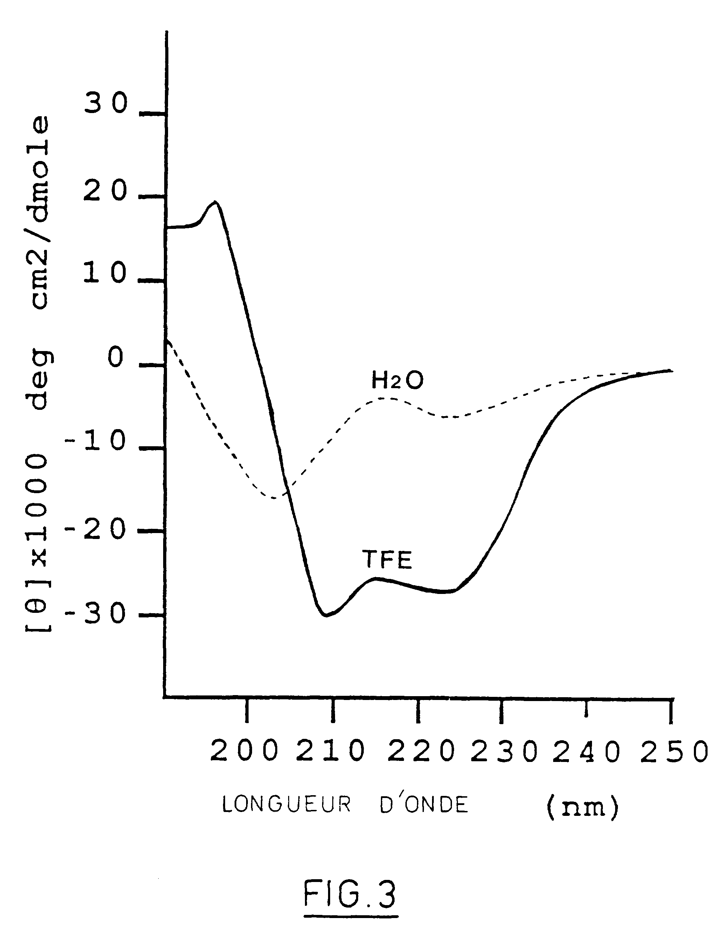

- la Fig. 3 représente les spectres de dichroïsme circulaire du peptide 89-122 dans l'eau et le trifluoroéthanol (TFE);

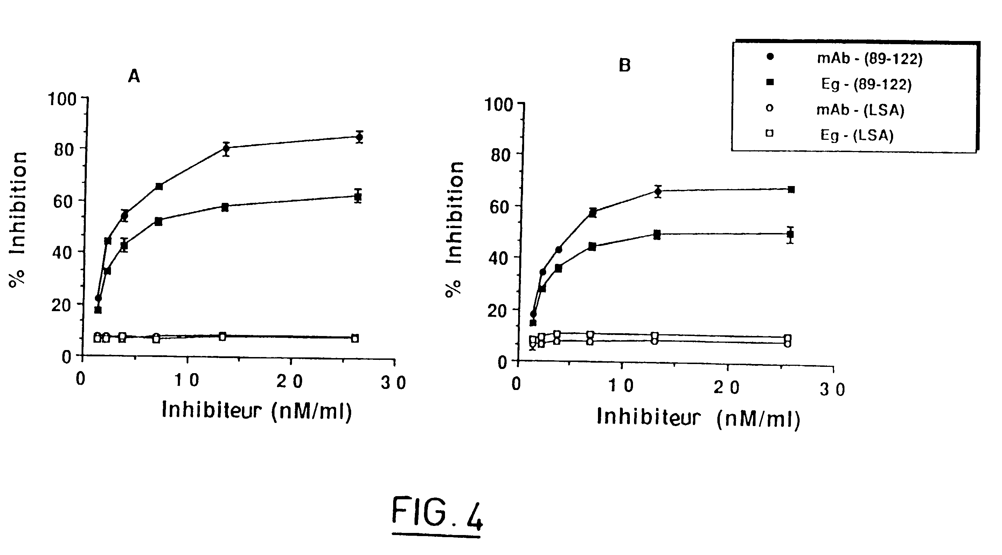

- la Fig. 4 représente les courbes d'inhibition de liaison de l'anticorps monoclonal Eg 0-2 154/12 (mAb) et de sérums humains de patients atteints d'hydatidose (Eg) à la protéine de fusion FP6 (A) ou aux antigènes de liquides de kystes hydatiques (B) par le peptide synthétique 89-122;

Un peptide correspondant à l'antigène du stade hépatique de l'infection par P. falciparum (LSA) a été utilisé en tant que témoin;

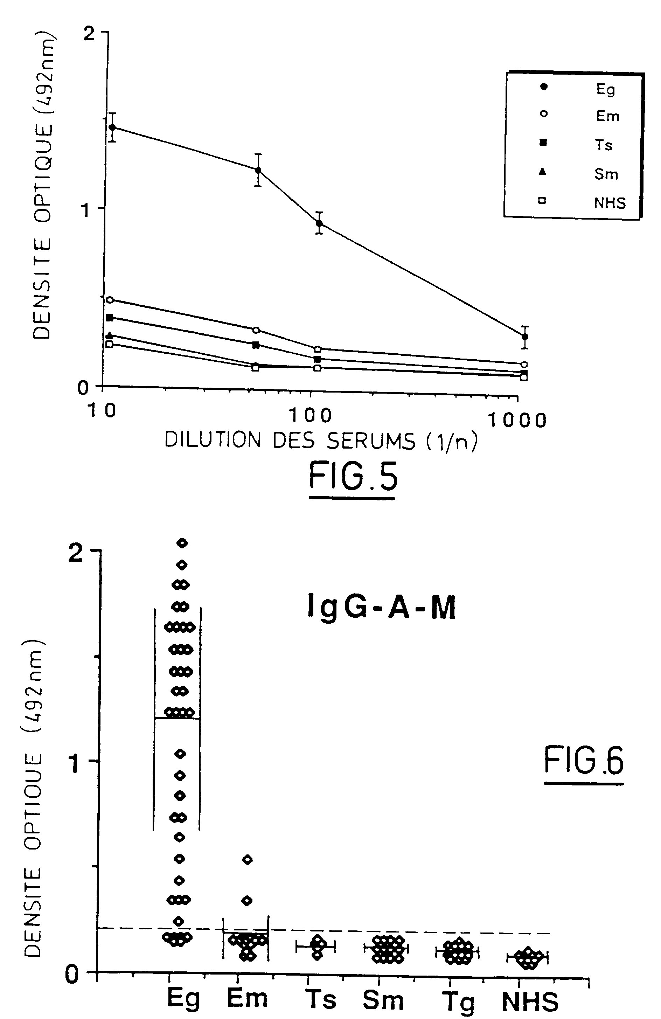

- la Fig. 5 représente la réactivité des IgG-A-M de sérums humains variés avec le peptide synthétique 89-122 utilisé sous forme d'antigène en phase solide dans un test ELISA. Les résultats représentent la moyenne ± l'écart type de 4 sérums humains différents : anti-E.granulosus (Eg), anti-E.mutilocularis (Em), anti-T.saginata (Ts), anti-S.mansoni (Sm), et des sérums humains normaux (NHS).
- la Fig. 6 représente la distribution de la réactivité d'IgG-A-M de sérums de patients infectés avec E.granulosus (Eg), E.multilocularis (Em), T.saginata (TS), S.mansoni (Sm), et T. gordii (Tg) et de sérums humains normaux (NHS) avec le peptide 89-122 en test ELISA. La ligne (---) représente la ligne de coupure déterminée à partir d'une moyenne obtenue à partir de sérums de témoins (individus non exposés habitant en France; N = 10) plus 3 valeurs de l'écart type;

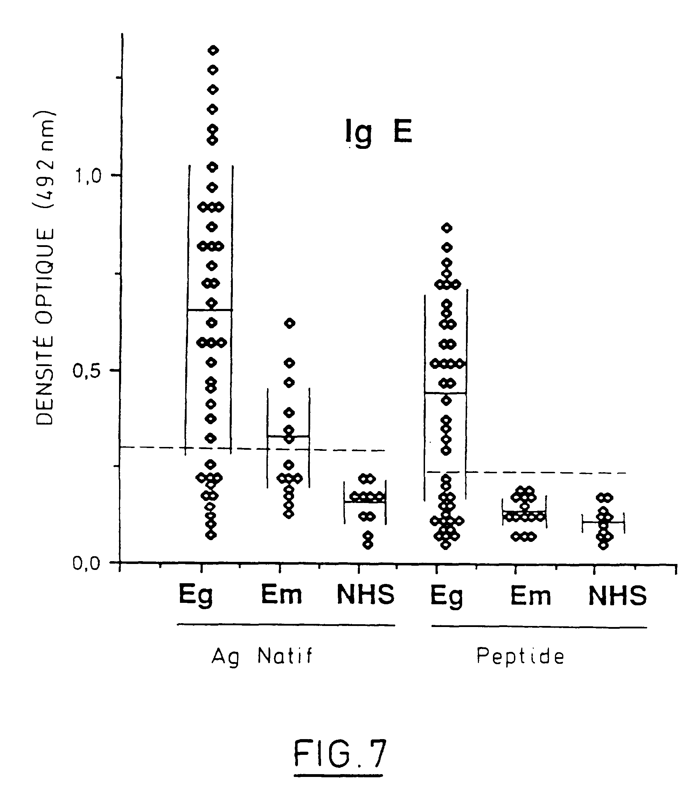

- la Fig. 7 représente la distribution de la réactivité d'IgE en test ELISA de sérums de patients infectés avec E.granulosus (Eg), E.multilocularis (Em), et de sérums humains normaux (NHS) avec le peptide 89-122 et des antigènes de liquide de kystes hydatiques (antigènes natifs), les valeurs au-dessus de la ligne (---) correspondant à la ligne de coupure (moyenne de 10 sérums témoins + 3 valeurs d'écart-type) ont été considérées comme positives.

### I/ Préparation des anticorps monoclonaux EG 02 154/12

Des souris BALB/C ont été immunisées par voie intrapéritonéale avec 100 µg d'une préparation antigénique (SHFAg) obtenue en ponctionnant aseptiquement des liquides hydatiques de mouton à partir de kystes hydatiques prélevés dans le foie et les poumons de l'animal qui ont été assemblés, ultracentrifugés à 40 000 tr/mn puis lyophilisés et repris dans de l'adjuvant de Freund complet (v/v).

Deux semaines après la dernière injection, une injection de rappel a été faite chez les souris avec 100 µg de la préparation antigénique SHF Ag en solution saline. Après 3 jours, les cellules de rate ont été récoltées, puis fusionnées avec des cellules de myélome SP 2/0 à un rapport de 1/5 dans du polyéthylène glycol (PEG 1500) selon la technique décrite par GALFRE et al (9). Les cellules ont été distribuées dans des puits sur des plaques de microculture et les cellules fusionnées sélectionnées dans un milieu HAT (Hypoxanthine Aminoptérine Thymidine). Les surnageants de culture de chaque puits montrant une croissance dense de cellules hybrides ont été testés pour la présence d'anticorps dirigé contre la préparation antigénique SHF Ag, par une méthode ELISA.

Parmi plusieurs anticorps monoclonaux réagissant positivement aux test ELISA avec la préparation antigénique SHF Ag, on a choisi une lignée cellulaire hybride (EG 02 154/12) produisant des anticorps de la sous-classe des Ig G₁. Contrairement au sérum de lapin dirigé contre une préparation antigénique SHF totale, l'anticorps monoclonal EG 02154/12 montrait, en immunoélectrophorèse une ligne de précipitation unique correspondant à l'antigène 5 tel que défini à partir de sérums hydatidiques humains de patients infectés par E. granulosus.

Aucune précipitation n'a été observée avec les antigènes de liquide de kystes d'E. multilocularis.

Les puits positifs ont été clonés par dilution limitante. Des liquides ascitiques de souris additionnés de cellules d'hybridome ont été fractionnés par précipitation au sulfate d'ammonium suivie d'une filtration sur gel de Trisacryl GF 05 (LKB, Suède). Les anticorps monoclonaux ont été purifiés par chromatographie d'échange d'ions sur DEAE-Trisacryl (LKB, Suède) et dialysés contre 10 mM de solution saline de tampon phosphate (PBS).

### II/ Obtention de l'ADN complémentaire

### 1. Obtention des sérums

Les sérums de patients ont été soumis aux tests classiques d'immunodiagnostic de l'échinococcose: immunoélectrophorèse (IEP) et test d'hémagglutination. Ils sont caractérisés vis-à-vis d'E.granulosus et d'E. multilocularis. La présence de l'arc 5 en IEP est déterminante pour le diagnostic d'infection à E.granulosus. Le diagnostic est par ailleurs confirmé par examen médical et intervention chirurgicale et/ou traitement thérapeutique.

### 2. Préparation des parasites

Le matériel parasitaire est récolté chez le mouton. Les kystes hydatiques sont prélévés dans le foie ou les poumons de l'animal et ponctionnés aseptiquement. Les protoscolex sont préparés par décantation du liquide recueilli. Le culot de sédimentation est repris, lavé plusieurs fois avec du liquide physiologique. Les scolex sont à chaque fois récupérés après sédimentation. Ils sont ensuite directement congelés dans un volume minimum d'azote liquide.

### 3. Extraction de l'ARN des protoscolex d'E.granulosus

L'ARN total est extrait des larves parasitaires selon un protocole modifié de la technique de CHIRGWIN J. et al (10). Les préparations de protoscolex d'E.granulosus stockées dans l'azote liquide sont broyées manuellement avant d'être reprises dans une solution de thiocyanate de guanidinium 4,2 M, N-lauroyl sarcosine 2%, EDTA pH8-10mM, 2-mercaptoéthanol 5% et acétate de sodium pH 4,5-20mM. Cette préparation est homogénéisée (Ultraturax) avant d'être soniquée et centrifugée à 10 000 tr/mn durant 20 mn (Sorvall-rotor HB-4). Le surnageant est ensuite déposé sur un coussin de 2 ml d'une solution de chlorure de césium (CsCl, densité = 1,72, EDTA pH8-10mM, acétate de sodium pH 5,5-50mM). Les tubes sont centrifugés durant 20 heures à 20°C, à 28 000 tr/mn (Beckman-rotor SW41). Le culot d'ARN est ensuite repris dans de l'eau et précipité à l'éthanol absolu à -20°C.

### 4. Préparation de la banque d'ADNc en vecteur phagique lambda gt11

A partir de 300 yg d'ARN total extrait des protoscolex d'E.granulosus, on synthétise le brin d'ADN complémentaire par l'action de la transcriptase reverse grâce à une amorce oligo-dT. L'ARN est ensuite attaqué par la ribonucléase H qui le perfore. Les morceaux d'ARN restants servent ensuite d'initiateurs à l'action de l'ADN polymérase I d'E. coli qui synthétise ainsi le second brin de l'ADNc. L'utilisation en phase finale de la T4 ADN polymérase permet de digérer les parties encore simple brin afin de créer un ADN complémentaire à bouts francs. Le protocole et les réactifs nécessaires à la synthèse de l'ADNc sont fournis par Amersham International (kit "cDNA synthesis system") ainsi que les enzymes pour le clonage en vecteur phagique. Les sites EcoRI de l'ADNc sont ensuite protégés par méthylation à l'aide de l'action de l'EcoRI méthylase. Des octanucléotides de synthèse double brin déphosphorylés (Amersham International) de séquence 5'-GGAATTCC-3' sont attachés aux extrémités franches de l'ADNc à l'aide de l'ADN ligase de T4. Les sites EcoRI des segments de liaison sont ensuite libérés par digestion par EcoRI. L'excès de segments de liaison est éliminé par chromatographie sur colonne (Amersham International). L'ADNc ainsi préparé est intégré dans le site EcoRI du phage λgt11 (YOUNG R. et DAVIS R. (11)) commercialisé par Promega Biotec sous la forme digérée par EcoRI et déphosphorylée. Après ligation, les phages sont encapsidés in vitro (système "Packagen in vitro packaging system" - Promega Biotec). La banque est ensuite testée par inoculation de la souche E. Coli Y1090 comme décrit par YOUNG R. et DAVIS R. (12).

### 5. Criblage de la banque et sélection de clones recombinants positifs

Un échantillon de cette banque en gt11 est inoculé sur la souche E. Coli Y1090 à une concentration de 5.10³ à 8.10³ ufp (unités formatrices de plaques)/boite de Pétri (90 mm de diamètre). L'expression de la protéine est sous le contrôle du promoteur lac et peut être induite par addition d'IPTG (Isopropyl-β-D-thiogalactopyranoside). L'IPTG est ajouté en plaçant sur les boites de Pétri, des filtres de nitrocellulose (Schleicher and Schuell) imprégnés d'IPTG 10 mM. Les protéines synthétisées sont adsorbées sur le filtre qui est ensuite incubé avec un mélange de 5 sérums de patients infestés par E.granulosus, destiné au criblage de la banque, à une dilution de 1/100 (à 4°C - pendant une nuit) en tampon PBS (Phosphate Buffer Saline : Na₂HPO₄ 10mM, NaH₂PO₄ 10mM, NaCl 150mM). Les sérums furent choisis en fonction de leurs caractéristiques immunoélectrophorétiques (de 3 à 6 arcs dont l'arc 5 en IEP classique). Les filtres sont ensuite lavés et les anticorps fixés sont reconnus par un second anticorps anti-immunoglobuline humaine (anti-IgG, A, M) marqué à la péroxidase (Pasteur Production - 1/500) et révélé par le réactif 4-chloro-1-naphtol (Bio Rad).

Sur les 1,5.10⁶ recombinants constituant la banque, environ 5.10⁵ phages ont été testés : 13 clones synthétisant une protéine ou une fraction de protéine portant des épitopes reconnus par le mélange de sérums de patients ont été sélectionnés. L'analyse électrophorétique en gel d'agarose a permis de mettre en évidence la taille des inserts d'ADNc pour chacun d'eux : clone 1 : un insert de 130 pb, clone 2 : 138 pb, clone 3 : 216 pb et pour les dix autres clones un insert de 456 pb.

Un second criblage de la banque a permis d'isoler un 14ème clone (Eg14) synthétisant une protéine ou une fraction de protéine contenant des épitopes reconnus par l'anticorps monoclonal EG 02152/14. L'analyse électrophorétique en gel d'agarose a montré que l'insert d'ADNc comportait 137 bp et présentait un seul cadre de lecture ouvert en phase avec λgt11, codant pour 37 aminoacides.

### 6. Test d'hybridation

Après clonage dans le site EcoRI du vecteur M13mp18, l'insert ADNc du clone 6(Eg 6 -456 pb) a été purifié et utilisé comme sonde lors d'un test d'hybridation.

Afin d'éviter tout problème de bruit de fond, l'insert d'ADNc utilisé comme sonde a été préalablement intégré dans le site EcoRI du vecteur M13mp18, puis purifié à partir d'un clone recombinant M13. La sonde (60-80ng) a été préparée et marquée à l'aide de ³PdCTP en utilisant la technique de FEINBERG et VOGELSTEIN (13). Les clones ont été cultivés et déposés sur la même boite de Pétri sous forme de goutte individualisant chacun d'entre eux. L'ADN a ensuite été adsorbé sur nitrocellulose directement à partir des plages de lyse et préparé selon le protocole de MASON et WILLIAMS (14).

L'hybridation a été réalisée selon les conditions décrites par WAHL et al (15), à 42°C en utilisant 5.10⁷ cpm de la sonde considérée. Les lavages ont été effectués dans des conditions de forte stringence à 65°C en tampon 0,1 x SSC, 0,1% SDS (SSC x 20 : NaCl 3M, Na₃citrate 2H₂O, ajusté à pH7), ceci afin de rechercher les homologies les plus strictes.

Aucun signal n'a été mis en évidence vis-à-vis des clones 1, 2 et 3, suggérant que ces derniers possèdent une séquence différente de l'insert 6. Par contre, les clones 4 à 13 apparaissent s'hybrider avec la sonde considérée suggérant des séquences similaires. Une analyse des séquences nucléotidiques a par ailleurs confirmé la stricte identité des clones 4 à 13.

### 7. Détermination de la séquence nucléotidique et protéique correspondante

L'ADN phagique total des clones est préparé selon la technique décrite par MANIATIS T. et al (16), puis digéré par EcoRI et analysé par électrophorèse sur gel d'agarose "low melting". Les inserts d'ADNc sont extraits par la technique du phénol chaud. Ils sont ensuite intégrés en vecteur phagique M13mp18 (Amersham International). Après transformation de la souche E.coli TG1, les matrices monobrins des vecteurs recombinants sont préparées. La techique de séquençage est basée sur le principe de la terminaison des chaînes par des didéoxynucléotides comme décrit par SANGER F. et al (17) et en utilisant des amorces photoluminescentes (Applied Biosystems). La fluorescence des fragments d'ADN est détectée par le séquenceur automatique 370 A DNA Sequencer (Applied Biosystems) et les séquences nucléotidiques sont ainsi déterminées comme décrit par SMITH L. et al (18). Les séquences protéiques correspondantes sont ensuite déduites par analyse informatique (PC Gene logiciel). Les homologies de séquences sont recherchées par ordinateur et consultation des banques de données Swissprot. (Switzerland) et NBRF (National Biomedical Research Fondation, Washington D.C.).

L'insert ADNc du clone 6 d'E.granulosus a été sous-cloné en vecteur phagique M13mp18 et la séquence nucléotidique a été déterminée (456 pb). Les extrémités 5' et 3' correspondent vraisemblablement à des sites internes EcoRI, non protégés lors de la réaction de méthylation de l'ADNc, où les séquences des segments de liaisons n'ont pas été retrouvées. Un seul cadre de lecture ouvert a été détecté, en phase avec le vecteur lambda, auquel correspond une chaîne polypeptitique de poids moléculaire calculé de 17,3 kDa. Les séquences nucléique et protéique ont été représentées précédemment. L'analyse par ordinateur indique une structure secondaire en hélice alpha (pour plus de 90%) du peptide considéré. Deux sites de glycolysation ont été trouvés, présents sur les acides aminés 72 et 84. Des homologies de séquences ont été recherchées par consultation des banques de données. Le taux d'homologie le plus élevé (20% sur 117 AA) a été obtenu par comparaison avec la chaîne lourde de la myosine du muscle squelettique de lapin (AA 100 à 216) et la chaîne lourde β de la myosine du muscle cardiaque de lapin (AA 508 à 620).

L'insert ADNc du clone 14 d'E. granulosus a été sous-cloné et séquencé selon un protocole strictement identique à celui utilisé pour le clone 6.

### 8. Construction lysogénique - Expression de la protéine de fusion

Des constructions lysogéniques sont obtenues en infectant des bactéries de la souche E.coli Y 1089 comme décrit par YOUNG R. & DAVIES (12) par 5 fois plus de phages gt11 du clone considéré. Cette souche bactérienne possède le répresseur lac (produit du gène lac I) qui inhibe l'expression de la protéine de fusion jusqu'à l'introduction d'IPTG dans le milieu. Elle permet en outre, de par ses caractéristiques (hfl A 150 (high frequency lysogeny)-sup F⁻), d'obtenir la phase lysogénique chez le phage. Les colonies lysogènes sont sélectionnées pour leur caractère de sensibilité à la température (le répresseur du phage devient non fonctionnel à 42°C). Les lysats bactériens contenant la protéine de fusion sont préparés selon le protocole décrit par HUYNT T. et al (19).

Après sélection des colonies lysogènes recombinantes pour le clone 6, l'expression de la protéine de fusion a été induite et les extraits lysogéniques préparés. L'analyse sur gel de polyacrylamide-SDS a montré un poids moléculaire apparent de 133kDa pour la protéine de fusion considérée (FP6), correspondant effectivement au poids moléculaire attendu de la fusion d'un polypeptide 152 AA avec la β-galactosidase.

### 9. Electrophorèse sur gel de polyacrylamide-SDS et immunoblotting

Les extraits lysogéniques sont analysés sur gel de polyacrylamide-SDS selon les conditions décrites par LAEMMLI U. - (21) en conditions réductrices en utilisant un gel de concentration de 3% et un gel de séparation de 7%. Les gels sont colorés après électrophorèse au bleu de Coomassie ou soumis à un électro-transfert selon les conditions de TOWBIN. et al (20). Après saturation en PBS-lait 3%, la nitrocellulose est incubée toute la nuit à 4°C avec des sérums de patients infestés par E.granulosus et E.multilocularis avec du PBS-lait 1%. Après lavages, un second anticorps spécifique marqué à la péroxydase est incubé (Pasteur Production - anti-Ig humaine 1/500, anti-IgG de souris 1/500). La révélation s'effectue dans les mêmes conditions que décrites précédemment au point 7 pour le criblage de la banque. Les sérums humains sont dilués en PBS de 1/100 à 1/500. L'anticorps monoclonal EG 02154/12 est dilué de 1/50 à 1/100 selon la concentration en immunoglobuline. Les sérums sont toujours préalablement incubés avec un extrait total de protéine E.coli ainsi qu'une solution de β-galactosidase purifiée (Sigma), afin de diminuer le bruit de fond.

Une dégradation protéolytique partielle a été observée lors de ces tests. Onze des 14 sérums humains anti-E.granulosus (soit 80% des sérums testés) reconnaissent la protéine recombinante de 133 kDa ainsi qu'une autre molécule de 81 kDa, de manière importante, correspondant vraisemblablement à un produit de dégradation. Une bande secondaire de 104 kDa a aussi été révélée de manière plus faible. Les mêmes bandes ont été détectées par 9 des sérums d'infection à E. multilocularis.

Par la suite, l'immunoréactivité de la protéine de fusion a été analysée vis-à-vis de l'anticorps monoclonal anti-antigène 5, EG 02 154/12. Cet anticorps a montré une reconnaissance de la protéine recombinante FP6 de 133 kDa, ainsi que des deux autres molécules de 104 kDa et 81 kDa, produits de dégradation de la première. Analysé dans les mêmes conditions, un sérum humain spécifique en IEP de l'antigène 5 (un arc = arc 5) a reconnu de la même manière les bandes de 133, 104 et 81 kDa. D'autre part, l'anticorps monoclonal EG 02 154/12 et les sérums d'infection ont été testés vis-à-vis des composants protéiques d'E. coli et la β-galactosidase purifiée, sans qu'aucune bande de masse moléculaire correspondant aux produits de dégradation ou à la protéine de fusion ait été révélée.

### 10. Synthèse des peptides.

Les peptides ont été synthétisés suivant une méthode discontinue en phase solide (22) dans un appareil automatique de synthèse de peptide (Applied Biosystems modèle 430a, Fester city, CA) suivant le protocole BOC-TFA (butoxycarbonyl-trifluoroacétate), sur une résine de type benzhydrylamine (Applied Biosystems). Les amino acides trifonctionnels ont été protégés de la manière suivante : Arg (Tos), Asp (OBzl), Glu (OBzl), Lys (2-ClZ (chlorobenzyloxy carbonyl)), Ser (OBzl). Les amino acides ont été introduits suivant une activation par un anhydride symétrique dans le DMF (couplage simple) sauf en ce qui concerne le Gln, qui a été introduit en utilisant le protocole d'activation DCC/HOBt (dicyclohexylcarbodiimide/butanol). La déprotection finale et le clivage des résines-peptidyles ont été réalisés selon un procédé à haute teneur en HF, pendant une heure à 0°C. Le peptide clivé déprotégé a été précipité avec de l'éther diéthylique froid puis dissous dans de l'acide acétique à 100°C et lyophilisé. Le peptide brut a été purifié par filtration sur gel (TSK HW40S, MERCK), suivie par une chromatographie HPLC en phase inverse sur une colonne Nucléosil C18, 300 Å 5,5 µ (Macherey Nagel), (12,7 mm x 500 mm) en utilisant un gradient à forte résolution sur 3 heures partant d'un tampon A1 (0,5‰ d'acide trifluoroacétique dans l'eau), jusqu'à 60% d'un tampon B1 (0,5‰d'acide trifluoroacétique/75% acétonitrile/25% eau) avec un débit de 2 ml/mm. La forme chlorhydrate du peptide a été obtenue en utilisant un gradient par étapes partant d'un tampon A2 (pH3 HCl dans l'eau) jusqu'à un tampon B2 (pH3 HCl 50%/isopropanol 50%) sur une colonne Nucléosil C18, 300 Å, 5 µ (12,7 mm x 75 mm). Les peptides ont été analysés en ce qui concerne leur homogénéité par HPLC analytique en phase inverse et en ce qui concerne leur identité par une analyse d'aminoacides et une détermination de masse moléculaire.

L'organisation hélicoïdale peut être mimée de manière appropriée par des peptides longs (Gras-Masse et coll. (24)).

Les séquences d'Eg6 et Eg14 ont été représentées en utilisant la technique Hydrophobic Cluster Analysis décrite par LEMESLE-VAROOT (23) (Fig. IB, IC). Cette technique ainsi que d'autres techniques plus classiques de prédiction de la structure secondaire, suggère l'organisation en forme de bande hélicoïdale. La distribution longitudinale du groupe hydrophobe le long de la bande hélicoïdale évoque fortement une structure enroulée d'hélice , telle que celle de la chaîne lourde de la myosine du muscle squelettique du lapin (Fig. 1.A).

A côté de leurs propriétés structurales communes, les clones Eg14 et Eg6 présentent des homologies de séquences limitées (Fig. 2). Cependant sur la représentation graphique, ces homologies de séquences discontinues forment des motifs topographiques présents sur les deux fragments qui peuvent expliquer la réactivité croisée observée avec Eg 02 154/12. En outre, la proportion élevée de résidus polaires ou chargées dans ces structures homologues peut plaider en faveur de leur présence dans des zones majoritairement exposées de l'antigène natif.

En conséquence, les inventeurs ont sélectionné et synthétisé un peptide de 34 aminoacides, reproduisant la séquence du clone Eg6 depuis l'amino acide 89 jusqu'à l'aminoacide 122 (Fig. 2) et rassemblant les deux motifs homologues (fig. 1B). Afin d'introduire des interactions de charges stabilisantes entre les extrémités de ce peptide désigné 89-122 et le dipole de l'hélice (Schoemaker et Coll. (25)), ils ont introduit à l'extrémité N-terminale un résidu d'acide glutamique chargé négativement et à l'extrémié C-terminale un résidu de lysine chargé positivement.

### 11. Etude de dichroïsme circulaire (CD)

Les spectres de CD ont été enregistrés sur un appareil ROUSSEL JOUAN 185 modèle II à température ambiante. Les concentrations de peptides ont été ajustées à partir de solutions mères par analyse quantitative d'aminoacides après hydrolyse acide totale. Les études de CD ont été réalisées sur les formes chlorhydrate du peptide, à une concentration de 10 mM dans NaCl 200 mM avec un trajet optique de 0,1 mm ou à une concentration de 1 mM dans du trifluoroéthanol 1mM avec un trajet optique de 1 mm. Les résultats de CD ont été exprimés en termes de structure hélicoïdale moyenne du résidu (θ) exprimé en deg.dmole⁻¹.cm. La teneur en hélices a été calculée à partir des spectres CD, en prenant (θ) 222 = -35,700 deg.dmole⁻¹.cm pour 100% de structure hélicoïdale.

L'organisation hélicoïdale du peptide a été vérifiée par des études de dichroïsme ciculaire, réalisées à température ambiante, dans une solution aqueuse ou aqueuse de trifluoroéthanol. Les résultats représentés sur la fig. 3 montrent que les deux spectres sont caractéristiques d'une organisation en hélice, avec des minima à 202-207 nm et 222-223 nm, et une valeur positive aux alentours de 290 nm. En prenant θ₂₂₂ = -35,700 deg.cm/dmole pour 100% de structure hélicoïdale, on a trouvé une proportion hélicoïdale de 14% en solution aqueuse et une proportion hélicoïdale de 74% dans du trifluoroéthanol aqueux.

### 12. Tests ELISA et d'inhibition.

Des plaques (Nunc) ont été recouvertes avec 100 µl d'Ag de liquide de kystes hydatiques ou d'extrait bactérien lysogène (FP6) à raison de 5 µg/ml de protéines. Les sérums humains ont été utilisés à une dilution au 1/100 et les anticorps liés ont été détectés à l'aide d'IgG-A-M anti-humain conjugés avec de l'HRP (peroxydase de raifort) (DIAGNOSTICS PASTEUR). Des sérums humains où l'anticorps EG 02 154/12 ont été préincubés pendant une nuit à 4°C avec différentes concentrations de peptides synthétiques. Le test ELISA avec des IgE a été réalisé avec des sérums humains dilués au 1/10 dans une solution saline de tampon phosphate et le peptide 89/122 a été étalé à raison d'1 µg par puits. Des IgG₁ de souris anti-IgE humains ont été obtenues par Southern Biotechnology Associates Inc. (Birmingham, Royaume-Uni). Les valeurs obtenues (moyenne ou doublet) ont été exprimées par la densité optique (DO) à 492 nm ± écart type (ET).

On donnera ci-après les résultats obtenus avec le peptide Eg 6 dans le diagnostic de l'hydatidose.

Le peptide 89-122 d'Eg 6 fixé directement sur des microplaques PVC est parfaitement reconnu par l'anticorps monoclonal EG 02 154/12 ainsi que par les sérums des patients infectés par E. granulosus dans un test ELISA comme l'indique la mesure des densités optiques sur le tableau I ci-après. Une bonne corrélation a été obtenue entre la reconnaissance de la protéine de fusion FP6 par les anticorps et leur fixation au peptide Eg 6.

**TABLEAU I**

| sérums et ascites dilués au 1/100 | moyenne des densités optiques | écart type |
|---|---|---|
| | ± | |
| sérums hydatiques reconnaissant FP6 (n = 25) | 1,96 | 0,57 |
| sérums hydatiques ne reconnaissant pas FP6 (n = 5) | 0,77 | 0,11 |
| sérums humains normaux (n = 5) | 0,22 | 0,07 |
| anticorps monoclonal (ascite EG 02 154/12) | 3,4 | 0,3 |
| ascite du myélome (SP2/0) | 0,12 | 0,04 |

D'autre part, on a mesuré le pouvoir inhibiteur de ce peptide dans la liaison de l'anticorps monoclonal EG 02 154/12 et de sérums humains de patients atteints d'hydatidose vis à vis de la protéine FP6 ou d'antigène de liquide de kystes hydatiques en test ELISA. Comme représenté sur la Fig. 4A, des inhibitions importantes de la liaison de l'anticorps monoclonal (80%) et de sérums humains de patients atteints d'hydatidose (60%) à la protéine FP6 ont été obtenues avec le peptide 89-122. Ce peptide est également capable d'inhiber de manière significative la liaision de ces anticorps aux antigènes de liquide de kystes hydatiques (65 et 45% respectivement (Fig. 4B)). L'inhibition de liaison est plus efficace pour l'anticorps monoclonal que pour les sérums humains de patients atteints d'hydatidose. Aucune inhibition significative n'est observée avec un peptide synthétique (26) non apparenté formant une hélice (41 aminoacides) correspondant au stade hépatique de l'infection par P.falciparum (27).

Ces résultats suggèrent que le peptide 89-122 est capable de mimer les sites de liaison reconnus spécifiquement par l'anticorps monoclonal Eg 02 154/12 et les sérums humains de patients atteint d'hydatidose.

On a également mesuré la réactivité de sérums de patients infectés par E.granulosus ou d'autres parasites vis à vis du peptide 89-122 utilisé comme antigène fixé, en test ELISA.

En utilisant différentes dilutions de sérums, on observe qu'une liaison significative à l'anticorps n'a lieu qu'avec des sérums anti-E.granulosus, comme représenté sur la Fig. 5. Les niveaux de liaison de sérums anti-E.multilocularis, anti-T.saginata, anti-S.mansoni étaient inférieurs aux valeurs obtenues avec des sérums humains normaux.

Dans des essais avec un peptide synthétique sans parenté, tous ces sérums ont montré des niveaux de réponse similaires.

Des tests ELISA ont également été réalisés pour démonter la valeur immunodiagnostique du peptide 82-122. Deux types de réponse anticorps humains (IgG-A-M et Ig-E) ont été analysés. En utilisant la limite supérieure des valeurs positives déterminée trois valeurs d'écart type au-dessus de la moyenne obtenue à partir du groupe témoin, on observe une sensibilité de liaison élevée (85%) et une bonne spécificité (86%) lors de la réponse IgG-A-M (Fig. 6). On a comparé ces résultats à ceux obtenus dans des essais de Western Blot avec FP6 et on a observé une amélioration de la spécificité, qui montre l'intérêt particulier 89-122 dans le diagnostic de l'hydatidose. Les résultats sont présentés dans le tableau II ci-après.

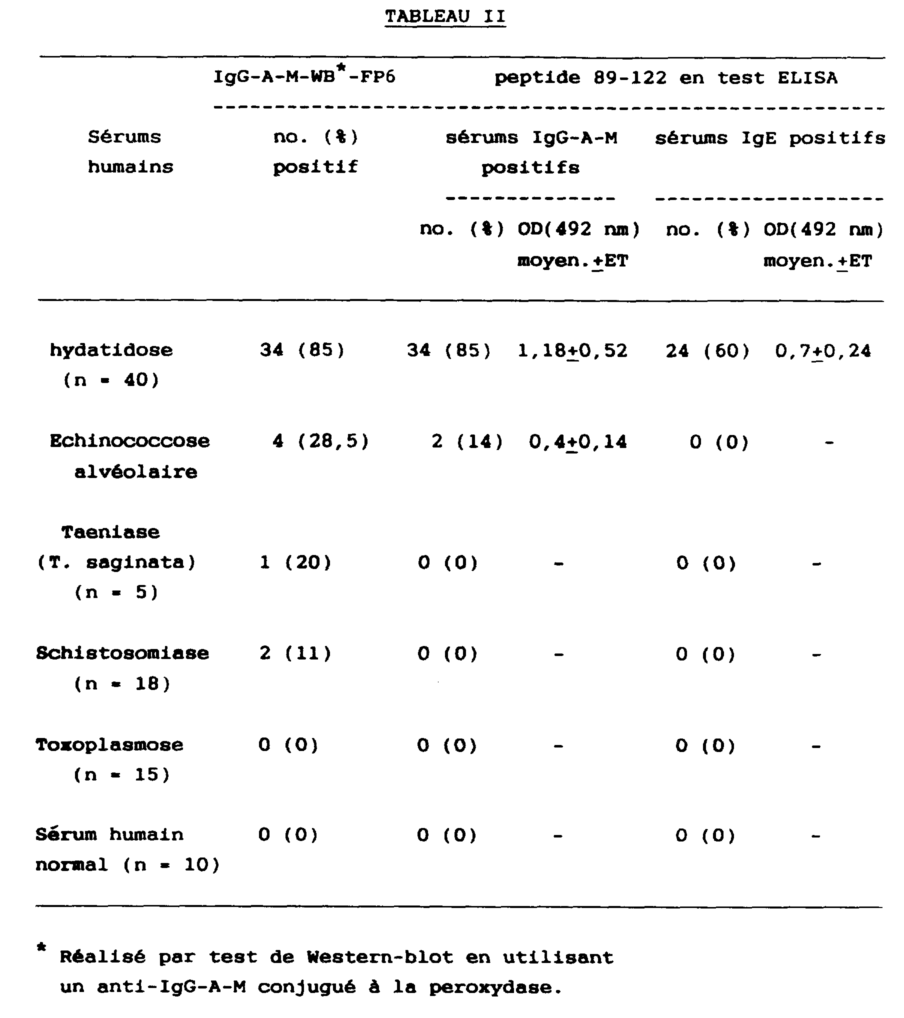

Comme le montre ce tableau, la réponse IgE vis à vis du peptide 89-122 montre une haute spécificité (100%) pour les patients infectés par E.granulosus. Aucune réactivité de type IgE n'a pu être détectée avec 52 sérums obtenus de patients atteints d'autres maladies parasitaires tandis que 60% des sérums humains des patients atteints d'hydatidose (24/40) se sont révélés positifs dans ce test IgE-ELISA. Un test témoin réalisé avec un peptide synthétique sans parenté (LSA) n'a montré aucune activité significative dans les deux tests ELISA.

La sensibilité et la spécificité de ce test ont été comparées aux tests ELISA-IgE réalisés avec des antigènes totaux de liquide hydatique (Fig. 7). La réactivité des IgE vis à vis du peptide 89-122 semble être plus spécifique (100%) pour E.granulosus tandis que 32% (6 sur 19) des sérums infectés par des cestodes ont montré une réaction croisée avec des antigènes de liquide de kystes hydatiques. Les anticorps IgE de 29 sur 40 (72% de patients infectés par E.granulosus) ont réagi avec les antigènes de liquide hydatique. La sensibilité du test ELISA-IgE observée avec le peptide 89-122 (60%) reste importante en comparaison de celle obtenue avec les antigènes hydatiques natifs.

| Symboles des acides aminés | | |
|---|---|---|
| | | |
| A | Ala | alanine |
| C | Cys | cystéine |
| D | Asp | acide aspartique |
| E | Glu | acide glutamique |
| F | Phe | phénylalanine |
| G | Gly | glycine |
| H | His | histidine |
| I | Ile | isoleucine |
| K | Lys | lysine |
| L | Leu | leucine |
| M | Met | méthionine |
| N | Asn | asparagine |
| P | Pro | proline |
| Q | Gln | glutamine |
| R | Arg | arginine |
| S | Ser | serine |
| T | The | thréonine |
| V | Val | valine |
| W | Trp | tryptophane |
| Y | Tyr | tyrosine |

### REFERENCES

1 - Capron, A., Biguet, J., Vernes, A. et Afchain D. (1968) Sructure antigénique des helminthes. Aspects immunologiques des relations hôte-parasite. Pathologie Biologie 16,121
2 - Russi S., Siracusano A., Remy G., Dropsy G. (1974) Isolation and characterization of blood P1 active carbohydrate antigen of Echinococcus granulosus cyst membrane. Journal of Immunology 112,1061
3 - Ben-Ismael, R., Carme, B., Niel G., Gentilini, M. (1980) Non-specific serological reactions with Echinococcus granulosus antigens. Rôle of anti-P1 antibodies. American Journal of Tropical Medicine and Hygiene 19,239
4 - CAPRON A., VERNES A & BIGUET J. (1967) Dans : Les Journées Lyonnaises d'hydatidologie (Ed. SIMEP), 27-40
5 - Capron A., Yarzabal L., Vernes A., Fruit J. (1970) Le diagnostic immunologique de l'echinococcose humaine. (bilan personnel à propos de 400 observations) Pathologie Biologie 18,357
6 - Oriol. R., Williams J.F., Peres Escandi M.V., Oriol (1971) Purification of lipoprotein antigens of Echinococcus granulosus from sheep hydatid fluid. American Journal of Tropical Medicine and Hygiene 20,569
7 - Bout, D., Fruit J., Capron, A. (1974) Purification d'un antigène spécifique du liquide hydatique. Annales de l'institut Pasteur (Immunology) 125C,775
8 - Piantelli M., Pozzuoli R., Arru E., Musiani P. (1977) Echinococcus granulosus : Identification of subunits of major antigens. Journal of Immunology 119,1382
9 - Galfre B.M., How S.C., Milstein C., Butcher G.W., Howard J.C. (1977) Antibodies to major histocompatibility antigens produced by hybrid cell lines. Nature 266,550
10 - CHIRGWIN J.M., PRZYBYLA A.E., MAC DONALD R.J. & RUTTER W.J. (1979) Biochem. 18 (24), 5294-5299
11 - YOUNG R.A. & DAVIS R.W. (1983) Proc. Natl. Acad. Sci. USA 80, 1194-1198
12 - YOUNG R.A. & DAVIS R.W. (1983) Science 22, 778-782
13 - FEINBERG A.P. & VOGELSTEIN B. (1983) Ann. Biochem 132, 6-13
14 - MASON P.J. & WILLIAMS J.G. (1985) In : A Practical Approach, Ed. B.D. Hames & S.I. Higgins,IRL Press, 113-137
15 - WAHL G.M., STERN M. & STARKG. R. (1979) Proc. Natl. Acad. Sci. USA 76(8), 3683-3687
16 - MANIATIS T., FRITSCH E.F. & SAMBROOK J. (1982) In: Molecular Cloning : A Laboratory Manual, Ed. Cold Spring Harbor Laboratory, 545.
17 - SANGER F., NICKLEN S. & COULSON A.R. (1977) Proc. Natl. Acad. Sci. USA 74 (12) 5463-5467
18 - SMITH L.M., SANDERS J. ZJ, KAISER R.J., HUGHES P., DOON C., CONNEL C.R., HEINER C., KENT S.B.H. & HOOD L.E. (1986) Nature 321, 674-679
19 - HUYNH T.V., YOUNG R.A. & DAVIS R.W. (1985) In : DNA Cloning Techniques (volume I ) : A Practical Approach, Ed. D.M. Glover, IRL Press, Oxford
20 - Towbin H., Staehelin T., Gordon J. (1979) Eletrophoretic transfer of proteins from polyacrylamide gels to nitrocellulose sheets : Procedure and some applications. Proceedings of the National Academy of Sciences (USA) 76, 4350
21 - Laemmli U.K. (1970) Cleavage of structural proteins during the assembly of the head of the bacteriphage T4. Nature 227, 680
22 - Merrifield, R.B. 1963. Solid-phase peptide synthetis. The synthesis of a tetrapeptide. J. Am. Chem. Soc. 85:2149-2155
23 - Lemesle-Varloot, L., B. Henrissat, C. Gaboriaud, V. Bissery, A. Morgat, and J.P. Mornon. 1990. Hydrophobic cluster analysis: procedures to derive structural and functional information from 2-D-representation of protein sequences. Biochimie. 72:555-574
24 - Gras-Masse H., M. Jolivet, H. Drobecq, J.F. Aubert, E.H. Beachey, F. Audibert, L. Chedid, and A. Tartar. 1988. Influence of helical organization on immunogenicity and antigenicity of synthetic peptides. Mol. Immunol. 25:673-678
25 - Shoemaker K. R., P.S. Kim, E.J. York, J.M. Stewart and R. L. Bladwin. 1987. Tests of the helix dipole model for stabilization of α-helices. Nature. 326:563-565
26 - Arturo Londono J., Gras-Masse H., C. Dubeaux, A. Tartar, and P. Druilhe. 1990. Secondary structure and immunogenicity of hybrid synthetic peptides derived from two Plasmodium falciparum pre-erythrocytic antigens. J. Immunol. 145:1557-1563
27 - Guerin, C., P. Druilhe, B. Galey, J. A. Londono, J. Patarapotikul, R.L. Beaudoin, A. Tartar, O. Mercereau-Puijalon and G. Langsley. 1987. A liver-stage-specific antigen of Plasmodium falciparum characterized by gene cloning. Nature 329:164-169

## Revendications

1. Séquence peptidique immunogène de l'antigène 5 d'Echinococcus granulosus, caractérisée en ce qu'elle est reconnue à la fois par des sérums de patients atteints d'hydatidose et par un anticorps monoclonal d'isotype IgG1 produit par l'hybridome EG 02154/12, déposé à la CNCM le 25 juin 1990 sous le n° 1-957.

2. Séquence peptidique immunogène selon la revendication 1, caractérisée en ce qu'elle comprend tout ou partie de la séquence : 
GluPheValAspIleAsnIleAlaSerLysValAlaAspAlaPheGlnLysAsnLysGlu

LysIleThrThrThrAspLysLeuGlyThrAlaLeuGluGlnValAlaSerGlnSerGlu

LysAlaAlaProGlnLeuSerLysMETLeuThrGluAlaSerAspValHisGlnArgMET

AlaThrAlaArgLysAsnPheAsnSerGluValAsnThrThrPheIleGluAspLeuLys

AsnPheLeuAsnThrThrLeuSerGluAlaGlnLysAlaLysThrLysLeuGluGluVal

ArgLeuAspLeuAspSerAspLysThrLysLeuLysAsnAlaLysThrAlaGluGlnLys

AlaLysTrpGluAlaGluValArgLysAspGluSerAspPheAspArgValHisGlnGlu

SerLeuThrIlePheGluLysThrCysLysGluPhe
 et les séquences qui en diffèrent par un ou plusieurs amino-acides et qui possèdent une activité immunogène analogue.

3. Séquence peptidique immunogène selon la revendication 2, comprenant une séquence choisie parmi :

EFVDINIASKVADAFQKNKEKITTT
ADAFQKNKEKITTTDKLGTALEQ
ITTTDKLGTALEQVASQSEKAAP
VASQSEKAAPQLSK
KAAPQLSKMLTEASDVHQRMATA
LSKMLTEASDVHQRMATARK
MATARKNFNSEVNTTFIEDLKNFLNTTLSEAQ
NFNSEVNTTFIEDLKNFLNTTLSEAQKAKTK
EAQKAKTKLEEVRLDLDSDKTKLKNAKTAEQKAK
AKTKLEEVRLDLDSDKTKLKNAK
EEVRLDLDSDKTKLKNAKTAEQKAKWEAEVRK
EQKAKWEAEVRKDESDFDRVHQESLTIFEKTCKEF
KDESDFDRVHQESLTIFEKTCKEF

4. Séquence peptidique immunogène selon la revendication 3, caractérisée en ce qu'elle est constituée par

EAQKAKTKLEEVRLDLDSDKTKLKNAKTAEQKAK

5. Séquence peptidique immunogène selon la revendication 1, caractérisée en ce qu'elle comprend tout ou partie de la séquence 
E F I R K Y D K G N K G K I N
L E E L T A M L D S V H R K T
S R A S M S R
 et les séquences qui en diffèrent par un ou plusieurs amino-acides et qui possèdent une activité immunogène analogue.

6. Séquence d'ADN comprenant une séquence nucléotidique codant pour une séquence peptidique selon l'une quelconque des revendications précédentes.

7. Séquence d'ADN codant pour une séquence peptidique selon l'une quelconque des revendications 1 à 4 comprenant essentiellement la séquence

10 20 30 40 50 60
GAATTCGTAGACATCAACATTGCATCTAAAGTCGCGGATGCTTTCCAGAAGAATAAGGAG

70 80 90 100 110 120
AAGATTACTACTACCGACAAACTGGGTACTGCTCTCGAGCAGGTTGCTTCCCAATCAGAA

130 140 150 160 170 180
AAGGCAGCTCCCCAACTTTCTAAAATGCTGACGGAAGCTTCTGATGTCCATCAGCGTATG

190 200 210 220 230 240
GCCACTGCCAGAAAGAATTTCAATAGTGAGGTTAATACCACCTTCATTGAAGATTTGAAA

250 260 270 280 290 300
AACTTCTTGAACACCACGCTTAGCGAGGCCCAGAAAGCAAAGACCAAGCTGGAGGAGGTT

310 320 330 340 350 360
CGACTAGATTTGGACTCTGACAAGACTAAATTGAAGAATGCTAAGACTGCGGAACAGAAG

370 380 390 400 410 420
GCCAAGTGGGAGGCCGAGGTGCGAAAAGACGAAAGTGACTTCGATCGAGTGCACCAAGAA

430 440 450
TCTCTTACTATCTTTGAGAAGACTTGCAAAGAATTC

8. Séquence d'ADN codant pour un peptide selon la revendication 1 ou 5 comprenant essentiellement la séquence

10 20 30 40
GAA TTC ATT CGA AAG TAT GAC AAG GGC AAT AAA GGC AAG ATC AAC
50 60 70 80 90
TTG GAA GAG TTG ACT GCT ATG CTC GAC AGT GTT CAT AGA AAA ACC
100 110 120 130
AGT AGA GCC TCA ATG AGC CGA TGA AGC ATT TAA AAT TAT GAG AAT

9. Plasmide comprenant une séquence d'ADN codant pour une séquence peptidique selon l'une quelconque des revendications 1 à 5.

10. Vecteur d'expression comprenant une séquence d'ADN codant pour une séquence peptidique selon l'une quelconque des revendications 1 à 5.

11. Hôte transformé avec un vecteur selon la revendication 10.

12. Procédé de diagnostic in vitro de l'hydatidose chez l'homme ou l'animal par mise en évidence des anticorps dirigés contre une séquence peptidique immunogène selon l'une quelconque des revendications 1 à 5 dans un prélèvement biologique de l'homme ou de l'animal dans lequel on met en contact la ou les séquences peptidiques immunogènes avec le prélèvement biologique de l'homme ou de l'animal pouvant contenir lesdits anticorps et on révèle la présence des anticorps fixés.

13. Procédé de diagnostic selon la revendication 12, caractérisé en ce qu'il consiste en une méthode radioimmunologique de type RIA, RIPA ou IRMA, ou une méthode immunoenzymatique de type Western-blot sur bandelettes ou de type ELISA.

14. Trousse de diagnostic in vitro de l'hydatidose pour la mise en oeuvre du procédé selon la revendication 12 ou 13, caractérisée en ce qu'elle comprend au moins une séquence peptidique selon l'une quelconque des revendications 1 à 5.

15. Trousse de diagnostic selon la revendication 14, caractérisée en ce qu'elle comprend en outre un anti-anticorps spécifique d'un isotype d'immunoglobuline.

16. Vaccin destiné au traitement et à la prévention de l'hydatidose chez l'homme ou chez l'animal, caractérisé en ce que l'agent vaccinant consiste en une séquence peptidique immunogène selon l'une quelconque des revendications 1 à 5.

17. Anticorps monoclonal dirigé contre une séquence peptidique immunogène selon l'une quelconque des revendications 1 à 5.

18. Anticorps monoclonal selon la revendication 17, caractérisé en ce qu'il est obtenu à partir de l'hybridome EG 02154/12 déposé à la CNCM le 25 juin 1990 sous le n° 1-957.

19. Hybridome EG 02154/12 produisant des anticorps monoclonaux de type IgG1 déposé à la CNCM le 25 juin 1990 sous le n° 1-957.

## Patentansprüche

1. Immunogene Peptidsequenz des Antigens 5 von Echinococcus granulosus, dadurch gekennzeichnet, daß sie unmittelbar durch Seren von Patienten, die von Hydatide befallen sind, und durch monoklonale Antikörper des Isotyps IgGl, hergestellt durch das Hybridom EG 02154/12, hinterlegt bei der CNCM am 25. Juni 1990 unter der Nr. 1-957, erkannt wird.

2. Immunogene Peptidsequenz nach Anspruch 1, dadurch gekennzeichnet, daß sie vollständig oder teilweise die Sequenz 
GluPheValAspIleAsnIleAlaSerLysValAlaAspAlaPheGlnLysAsnLys
GluLysIleThrThrThrAspLysLeuGlyThrAlaLeuGluGlnValAlaSerGln
SerGluLysAlaAlaProGlnLeuSerLysMETLeuThrGluAlaSerAspValHis
GlnArgMETAlaThrAlaArgLysAsnPheAsnSerGluValAsnThrThrPheIle
GluAspLeuLysAsnPheLeuAsnThrThrLeuSerGluAlaGlnLysAlaLysThr
LysLeuGluGluValArgLeuAspLeuAspSerAspLysThrLysLeuLysAsnAla
LysThrAlaGluGlnLysAlaLysTrpGluAlaGluValArgLysAspGluSerAsp
PheAspArgValHisGlnGluSerLeuThrIlePheGluLysThrCysLysGluPhe
 umfaßt und die Sequenzen, die davon durch eine oder mehrere Aminosäuren abweichen und die eine analoge immunogene Wirkung besitzen.

3. Immunogene Peptidsequenz nach Anspruch 2, umfassend eine Sequenz, ausgewählt aus

EFVDINIASKVADAFQKNKEKITTT
ADAFQKNKEKITTTDKLGTALEQ
ITTTDKLGTALEQVASQSEKAAP
VASQSEKAAPQLSK
KAAPQLSKMLTEASDVHQRMATA
LSKMLTEASDVHQRMATARK
MATARKNFNSEVNTTFIEDLKNFLNTTLSEAQ
NFNSEVNTTFIEDLKNFLNTTLSEAQKAKTK
EAQKAKTKLEEVRLDLDSDKTKLKNAKTAEQKAK
AKTKLEEVRLDLDSDKTKLKNAK
EEVRLDLDSDKTKLKNAKTAEQKAKWEAEVRK
EQKAKWEAEVRKDESDFDRVHQESLTIFEKTCKEF
KDESDFDRVHQESLTIFEKTCKEF.

4. Immunogene Peptidsequenz nach Anspruch 3, dadurch gekennzeichnet, daß sie besteht aus

EAQKAKTKLEEVRLDLDSDKTKLKNAKTAEQKAK.

5. Immunogene Peptidsequenz nach Anspruch 1, dadurch gekennzeichnet, daß sie vollständig oder teilweise die Sequenz 
E F I R K Y D K G N K G K I N
L E E L T A M L D S V H R K T
S R A S M S R
 umfaßt und die Sequenzen, die davon in einer oder mehreren Aminosäuren abweichen und die eine analoge immunogene Wirkung besitzen.

6. DNA-Sequenz, enthaltend ein Nukleotid, das für eine Peptidsequenz nach einem der vorhergehenden Ansprüche kodiert.

7. DNA-Sequenz, die für eine Peptidsequenz nach einem der vorangegangenen Ansprüche 1 bis 4 kodiert und im wesentlichen die Sequenz 
10 20 30 40 50
GAATTCGTAGACATCAACATTGCATCTAAAGTCGCGGATGCTTTCCAGAA
60 70 80 90 100
GAATAAGGAGAAGATTACTACTACCGACAAACTGGGTACTGCTCTCGAGC
110 120 130 140 150
AGGTTGCTTCCCAATCAGAAAAGGCAGCTCCCCAACTTTCTAAAATGCTG
160 170 180 190 200
ACGGAAGCTTCTGATGTCCATCAGCGTATGGCCACTGCCAGAAAGAATTT
210 220 230 240 250
CAATAGTGAGGTTAATACCACCTTCATTGAAGATTTGAAAAACTTCTTGA
260 270 280 290 300
ACACCACGCTTAGCGAGGCCCAGAAAGCAAAGACCAAGCTGGAGGAGGTT
310 320 330 340 350
CGACTAGATTTGGACTCTGACAAGACTAAATTGAAGAATGCTAAGACTGC
360 370 380 390 400
GGAACAGAAGGCCAAGTGGGAGGCCGAGGTGCGAAAAGACGAAAGTGACT
410 420 430 440 450
TCGATCGAGTGCACCAAGAATCTCTTACTATCTTTGAGAAGACTTGCAAA
GAATTC
 umfaßt.

8. DNA-Sequenz, die für ein Peptid nach Anspruch 1 oder 5 kodiert, im wesentlichen umfassend die Sequenz

10 20 30 40
GAA TTC ATT CGA AAG TAT GAC AAG GGC AAT AAA GGC AAG ATC

50 60 70 80
AAC TTG GAA GAG TTG ACT GCT ATG CTC GAC AGT GTT CAT AGA

90 100 110 120
AAA ACC AGT AGA GCC TCA ATG AGC CGA TGA AGC ATT TAA AAT

130
TAT GAG AAT.

9. Plasmid, enthaltend eine DNA-Sequenz, die für eine Peptidsequenz nach einem der Ansprüche 1 bis 5 kodiert.

10. Expressionsvektor, enthaltend eine DNA-Sequenz, die für eine Peptidsequenz nach einem der Ansprüche 1 bis 5 kodiert.

11. Transformierter Wirt mit einem Vektor nach Anspruch 10.

12. Verfahren zur in vitro-Diagnose der Hydatide beim Menschen oder beim Tier durch Nachweis von Antikörpern, die gegen eine immunogene Peptidsequenz nach einem der Ansprüche 1 bis 5 in einer dem Menschen oder dem Tier entnommenen biologischen Probe gerichtet sind, indem man die immunogene(n) Peptidsequenz(en) mit der dem Menschen oder dem Tier entnommenen biologischen Probe, die besagte Antikörper enthalten kann, in Kontakt bringt und das Vorhandensein von fixierten Antikörpern ermittelt.

13. Diagnoseverfahren nach Anspruch 12, dadurch gekennzeichnet, daß es aus einer radioimmunologischen Methode des Typs RIA, RIPA oder IRMA oder einer immunoenzymatischen Methode vom Typ des Western-Blots auf Bändchen oder vom Typ eines ELISA besteht.

14. Diagnostische Einrichtung zur in vitro-Diagnose der Hydatide zum Einsatz in einem Verfahren nach den Ansprüchen 12 oder 13, dadurch gekennzeichnet, daß sie wenigstens eine Peptidsequenz gemäß den Ansprüchen 1 bis 5 umfaßt.

15. Diagnostische Einrichtung nach Anspruch 14, dadurch gekennzeichnet, daß sie ferner einen Antikörper gegen einen Antikörper umfaßt, der spezifisch für einen Isotyp des Immunoglobulins ist.

16. Impfstoff zur Behandlung und Vorbeugung der Hydatide beim Menschen oder beim Tier, dadurch gekennzeichnet, daß das Impfmaterial aus einer immunogenen Peptidsequenz nach einem der Ansprüche 1 bis 5 besteht.

17. Monoklonaler Antikörper gegen eine immunogene Peptidsequenz nach einem der Ansprüche 1 bis 5.

18. Monoklonaler Antikörper nach Anspruch 17, dadurch gekennzeichnet, daß er ausgehend vom Hybridom EG 02154/12, hinterlegt bei der CNCM am 25. Juni 1990 unter der Nr. 1-957, erhalten wird.

19. Hybridom EG 02154/12, welches die monoklonalen Antikörper des Typs EgG1, hinterlegt bei der CNCM am 25. Juni 1990 unter der Nr. 1-957, erzeugt.

## Claims

1. Immunogenic peptide sequence of antigen 5 of Echinococcus granulosus, characterised in that it is recognised by both the serums of patients infected by hydatid disease and by a monoclonal antibody of Ig G1 isotype produced by the hybridoma EG 02154/12 which was deposited at the CNCM as No. 1-957 on 25 June 1990.

2. Immunogenic peptide sequence according to Claim 1, characterised in that it comprises all or part of the sequence: 
GluPheValAspIleAsnIleAlaSerLysValAlaAspAlaPheGlnLysAsnLysGlu

LysIleThrThrThrAspLysLeuGlyThrAlaLeuGluGlnValAlaSerGlnSerGlu

LysAlaAlaProGlnLeuSerLysMETLeuThrGluAlaSerAspValHisGlnArgMET

AlaThrAlaArgLysAsnPheAsnSerGluValAsnThrThrPheIleGluAspLeuLys

AsnPheLeuAsnThrThrLeuSerGluAlaGlnLysAlaLysThrLysLeuGluGluVal

ArgLeuAspLeuAspSerAspLysThrLysLeuLysAsnAlaLysThrAlaGluGlnLys

AlaLysTrpGluAlaGluValArgLysAspGluSerAspPheAspArgValHisGlnGlu

SerLeuThrIlePheGluLysThrCysLysGluPhe
 and the sequences differing therefrom by one or more amino acids and having an analogous immunogenic activity.

3. Immunogenic peptide sequence according to Claim 2, comprising a sequence selected from:

EFVD IN IASKVADAFQKNKEKITTT

ADAFQKNKEKITTTDKLGTALEQ

ITTTDKLGTALEQVASQSEKAAP

VASQSEKAAPQLSK

KAAPQLSKMLTEASDVHQRMATA

LSKMLTEASDVHQRMATARK

MATARKNFNSEVNTTF IEDLKNFLNTTLSEAQ

NFNSEVNTTF IEDLKNFLNTTLSEAQKAKTK

EAQKAKTKLEEVRLDLDSDKTKLKNAKTAEQKAK

AKTKLEEVRLDLDSDKTKLKNAK

EEVRLDLDSDKTKLKNAKTAEQKAKWEAEVRK

EQKAKWEAEVRKDESDFDRVHQESLT IFEKTCKEF

KDESDFDRVHQESLT IFEKTCKEF

4. Immunogenic peptide sequence according to Claim 3, characterised in that it is constituted by

EAQKAKTKLEEVRLDLDSDKTKLKNAKTAEQKAK

5. Immunogenic peptide sequence according to Claim 1, characterised in that it comprises all or part of the sequence 
E F I R K Y D R G N R G K I N
L E E L T A M L D S V H R R T
S R A S H S R
 and the sequences differing therefrom by one or more amino acids and having an analogous immunogenic activity.

6. DNA sequence comprising a nucleotide sequence coding for a peptide sequence according to any of the preceding claims.

7. DNA sequence coding for a peptide sequence according to any of Claims 1 to 4 and substantially comprising the sequence

10 20 30 40 50 60
GAATTCGTAGACATCAACATTGCATCTAAAGTCGCCGATGCTTTCCAGAAGAATAACGAC

70 80 90 100 110 120
AAGATTACTACTACCGACAAACTGGGTACTGCTCTCGAGCAGGTTGCTTCCCAATCAGAA

130 140 150 160 170 180
AAGGCAGCTCCCCAACTTTCTAAAATGCTGACGGAAGCTTCTGATGTCCATCAGCGTATG

190 200 210 220 230 240
GCCACTGCCAGAAAGAATTTCAATAGTGAGGTTAATACCACTTTCATTGAAGATTTGAAA

250 260 270 280 290 300
AACTTCTTGAACACCACGCTTAGCGAGCCCAGAAAGCAAAGACCAAGCTGGAGGAGGTT

310 320 330 340 350 360
CGACTAGATTTGGACTCTGACAAGACTAAATTGAAGAATGCTAAGACTCCGGAACAGAAG

370 380 390 400 410 420
GCCAAGTGGGAGGCCGAGGTGCGAAAAGACGAAAGTGACTTCGATCGAGTGCACCAAGAA

430 440 450
TCTCTTACTATCTTTGAGAAGACTTGCAAAGAATTC

8. DNA sequence coding for a peptide according to Claim 1 or 5 and substantially comprising the sequence

10 20 30 40
GAA TTC ATT CGA AAG TAT GAC AAG GGC AAT AAA GGC AAG ATC AAC

50 60 70 80 90
TTG GAA GAG TTG ACT GCT ATG CTC GAC AGT GTT CAT AGA AAA ACC

100 110 120 130
AGT AGA GCC TCA ATG AGC CGA TCA AGC ATT TAA AAT TAT GAG AAT

9. Plasmid comprising a DNA sequence coding for a peptide sequence according to any of Claims 1 to 5.

10. Expression vector comprising a DNA sequence coding for a peptide sequence according to any of Claims 1 to 5.

11. Most transformed by a vector according to Claim 10.

12. Process for the in vitro diagnosis of hydatid disease in man or in animals by means of showing up antibodies directed against an immunogenic peptide sequence according to one of Claims 1 to 5 in a biological sample provided by the man or the animal, in which the immunogenic peptide sequence or sequences is or are brought into contact with the biological sample provided by the man or the animal, which may contain the said antibodies, and the presence of the fixed antibodies is revealed.

13. Process for diagnosis according to Claim 12, characterised in that it comprises a radioimmunological method of the RIA, RIPA or IRMA type, or an immunoenzymatic method of the Western blot type on strips or of the ELISA type.

14. Diagnostic kit for the in vitro diagnosis of hydatid disease for carrying out the process according to Clain 12 or 13, characterised in that it comprises at least one peptide sequence according to any of Claims 1 to 5.

15. Diagnostic kit according to Claim 14, characterised in that it additionally comprises a specific anti-antibody of an immunoglobulin isotype.

16. Vaccine intended for the treatment and prevention of hydatid disease in man or in animals, characterised in that the vaccination agent comprises an immunogenic peptide sequence according to any of Claims 1 to 5.

17. Monoclonal antibody directed against an inmunogenic peptide sequence according to any of Claims 1 to 5.

18. Monoclonal antibody according to claim 17, characterised in that it is obtained from the hybridoma EG 02154/12 which was deposited at the CNCM as No. 1-957 on 25 June 1990.

19. The hybridoma EG 02154/12 producing monoclonal antibodies of the IgG1 type, which was deposited at the CNCM as No. 1-937 on 25 June 1990.
